# EUROPEAN PATENT SPECIFICATION

(11) **EP 0 789 561 B1**
(45) Date of publication and mention of the grant of the patent: **07.04.2004**
(21) Application number: 96926214.6
(22) Date of filing: 31.07.1996
(51) Int. Cl.: A61K 9/48, A61K 47/34

(54) **ORAL CYCLOSPORIN FORMULATIONS**
ORALE CYCLOSPORINFORMULIERUNGEN
COMPOSITIONS ORALES DE CYCLOSPORINE

(30) Priority: 25.08.1995 US 519689; 21.03.1996 US 620021; 25.03.1996 US 622516
(43) Date of publication of application: 20.08.1997
(73) Proprietor: SANGSTAT MEDICAL CORPORATION, Menlo Park, CA 94025 (US); UNIVERSITY OF NORTH CAROLINA AT CHAPEL HILL, Chapel Hill, NC 27599-4105 (US)
(72) Inventor: CHO, Moo J., Chapel Hill, NC 27514 (US); LEVY, Ralph E., Pleasanton, CA 94566 (US); POULETTY, Philippe J., Atherton, CA 94027 (US); FLOC'H, Robert, 44300 Nantes (FR); MERLE, Christian, 86000 Poitiers (FR)
(74) Representative: Baldock, Sharon Claire
(86) International application number: PCT/US1996/012569
(87) International publication number: WO 1997/007787

(56) References cited:
- EP-A- 0 589 843
- WO-A-92/09299
- CA-A- 2 106 827
- DE-A- 4 340 781
- DE-A- 19 539 860
- GB-A- 2 015 339
- GB-A- 2 221 157
- Fiedler, Lexikon der Hilfsstoffe für Pharmazie, Kosmetik und angrenzende Gebiete, vol. 2, 1989, p. 984f.

## Description

### INTRODUCTION

### Field of the Invention

The field of this invention is oral cyclosporin formulations.

### Background

Despite efforts to avoid graft rejection through host-donor tissue type matching, in the majority of transplantation procedures where a donor organ is introduced into a host, immunosuppressive therapy is critical to the maintained viability of the donor organ in the host. A variety of immunosuppressive agents have been employed in transplantation procedures, including azathioprine, methotrexate, cyclophosphamide, FK-506, rapamycin and corticosteroids. Agents finding increased use in immunosuppressive therapy due to their preferential effect on T-cell mediated reactions are the cyclosporins.

Cyclosporins are a class of cyclic polypeptides consisting of eleven amino acids which are produced as a metabolite by the fungus species Tolypocladium inflatum Gams. Cyclosporins have been observed to reversibly inhibit immunocompetent lymphocytes, particularly T-lymphocytes, in the G₀ or G₁ phase of the cell cycle. Cyclosporins have also been observed to reversibly inhibit lymphokine production and release. Although a number of cyclosporins are known, Cyclosporin A is the most widely used.

Use of Cyclosporin A has been reported to prolong the survival of allogeneic transplants involving skin, heart, kidney, pancreas, bone marrow, small intestine and lung. In allogeneic transplantations, Cyclosporin A has been shown to suppress humoral immunity and, to a greater extent, cell mediated immune reactions, including: allograft rejection, delayed hypersensitivity, experimental allergic encephalomyelitis, Freund's adjuvant arthritis, and graft vs. host disease. Although success has been realized with Cyclosporin A, following transplantation administration of the agent must be continued since the benefits of cyclosporin therapy are reversible and graft rejection occurs once administration of Cyclosporin A is discontinued.

Although cyclosporin formulations for both oral and intravenous administration have been developed, oral administration of cyclosporin is preferred because of the ease of administration and greater patient acceptance. Furthermore, intravenous administration of cyclosporin can result in anaphylactic reactions, a side effect not observed with oral formulations. Oral cyclosporin formulations which have been developed and are currently marketed include both soft gelatin capsule and solution formulations, both of which are sold under the trademarks SANDIMMUNE® and NEORAL™.

In using oral cyclosporin formulations in immunosuppressive therapy, both the care giver and manufacturer must be cognizant of many issues. With oral cyclosporin formulations, cyclosporin bioavailability can be limited because of cyclosporin's immiscibility in water and the tendency of cyclosporin to precipitate in aqueous environments. In addition, the concentration of cyclosporin present in oral formulations can be limited due to cyclosporin's hydrophobic nature. Furthermore, cyclosporin absorption by the gastrointestinal tract can be erratic from one formulation batch to the next, requiring constant monitoring of cyclosporin blood levels during treatment. Finally, packaging and storage stability are an issue with oral formulations. For example, with soft gelatin capsule formulations of cyclosporin, air tight packaging must be employed, which is inconvenient due to bulkiness and high cost. Furthermore, cyclosporine formulations may be unstable at lower temperatures, as cyclosporine crystallization may occur.

Thus, desirable oral cyclosporin formulations would be formulations that address at least some of the above issues. Ideally, oral formulations would promote high bioavailability, comprise high concentrations of cyclosporin and would be amenable to preparation in hard capsule form:

### Relevant Literature

Physician's Desk Reference (1994) pp 2071-2074 describes oral cyclosporin formulations currently sold under the trademark SANDIMMUNE®.

Oral cyclosporine formulations are also described in the NEORAL™ package insert, (1995) (Sandoz Pharmaceuticals Corporation, East Hanover, New Jersey, 07936).

EP-A-0 589 843 discloses a pharmaceutical composition in the form of an emulsion preconcentrate for oral administration and containing a cyclosporin. The pharmaceutical composition has a carrier medium for the cyclosporin that contains a hydrophilic organic solvent ; a mixed mono-, di-, and tri-glyceride or a transesterified and polyethoxylated vegetable oil ; and a polyoxyethylene-sorbitan-fatty acid ester surfactant. The pharmaceutical composition provides high bioavailability and low inter- and intra-subject variability.

U.S. Patents of interest describing cyclosporins and derivatives thereof include: 4,220,641; 4,639,434; 4,289,851; and 4,384,996. U.S. Pat. No. 5,047,396 describes an intravenous preparation for administration of cyclosporin. U.S. Pat. Nos. 4,388,307; 4,970,076 and 4,990,337 describe the preparation of oral cyclosporin formulations.

The preparation of hard capsules for the oral delivery of pharmaceutical formulations is described in U.S. Pat. Nos. 4,822,618; 4,576,284; 5,120,710; and 4,894,235.

### SUMMARY OF THE INVENTION

Oral cyclosporin formulations, and methods for their use in immunosuppressive therapy, are provided. In the subject formulations, cyclosporin is present in an orally acceptable vehicle comprising at least one alkanol solvent of from 2 to 3 carbon atoms in combination with at least one non-ionic surfactant. The subject formulations may further comprise one or more cosolvents, where cosolvents of interest are fatty acid esters and diols. The cyclosporin formulations can be packaged as hard capsules. By including a polyoxyalkylene surfactant, upon diluting of the stable dispersion into an aqueous medium; amorphous bioavailable cyclosporin nanoparticles are provided.

The present invention is as defined by the appended claims.

### BRIEF DESCRIPTION OF THE FIGURES

Fig. 1 provides the cyclosporin peak concentration (Cₘₐₓ) achieved in rats for several oral formulations according to the subject invention, where the Cₘₐₓ is shown as a relative value compared to the Cₘₐₓ achieved with SANDIMMUNE® ORAL formulation (SO).
Fig. 2 provides the time at which Cₘₐₓ occurred (Tₘₐₓ) for each of formulations shown in Fig. 1, where Tₘₐₓ is provided as relative value compared to the Tₘₐₓ of SANDIMMUNE® ORAL formulation (SO).
Fig. 3 provides the relative area under the blood concentration-time curve (AUC) for each of the formulations shown in Fig. 1, where AUC is provided as a relative value compared to the AUC value for SANDIMMUNE® ORAL formulation (SO).
Fig. 4 provides the cyclosporin peak concentration (Cₘₐₓ) achieved in humans for several oral formulations according to the subject invention, as well as SANDIMMUNE® ORAL solution ("Sand" in the figure).
Fig. 5 provides the time at which Cₘₐₓ occurred (Tₘₐₓ) for each of formulations shown in Fig. 4.
Fig. 6 provides the area under the blood concentration-time curve (AUC) for each of the formulations shown in Fig. 4.

### DESCRIPTION OF THE SPECIFIC EMBODIMENTS

Oral cyclosporin formulations are provided which promote bioavailability and can be formulated as capsules, particularly hard capsules. In the subject formulations, cyclosporin is present in an orally acceptable vehicle comprising at least one alkanol solvent of from 2 to 3 carbon atoms in combination with at least one non-ionic surfactant. The subject formulations may further comprise at least one cosolvent, where cosolvents of interest include fatty acid esters and diols. Each of the components of the subject formulations are pharmaceutically acceptable. In addition to providing for high bioavailability, the subject formulations provide for reproducible cyclosporin absorption from one batch of a particular formulation to the next. The subject formulations find use in immunosuppressive therapy.

A number of cyclosporins are known in the art to exhibit immunosuppressive activity and may be delivered in the subject oral formulations. Cyclosporins that may be administered in the subject formulations include Cyclosporin A, Cyclosporin B, Cyclosporin C, Cyclosporin D and Cyclosporin G, as well as synthetic analogs thereof. *See* Merck Index (1989) 2759. The subject oral formulations are particularly suited for the delivery of Cyclosporin A. When delivered in the subject formulations, Cyclosporin A will be present in concentrations ranging from 50 to 150 mg/ml, usually 100 to 150 mg/ml, based on the volume of the vehicle component of the formulation.

The vehicle component of the subject formulations will include an alkanol solvent component, where the alkanol solvent component will comprise at least one alkanol and usually no more than three different alkanols, more usually no more than two different alkanols; where the alkanols will usually be from 2 to 3 carbon atoms, and from 1 to 2 hydroxy groups, such that there is no more than 1 hydroxy group per 1.5 carbon atoms. Suitable alkanols include ethanol and propylene glycol. The total amount of alkanol solvent in the formulation will be at least about 1 % (v/v), usually at least about 3 % (v/v) and may be as high as 95 % (v/v), but will generally range from about 5 to 75 % (v/v), usually from about 5 to 60 % (v/v), and more usually from about 10 to 60% (v/v) of the formulation. When ethanol is present in the formulation as an alkanol solvent, the amount of ethanol may range from 5 to 20 % (v/v), usually from about 5 to 15 % (v/v) of the formulation, while when propylene glycol is present as an alkanol solvent, the amount of propylene glycol in the subject formulation may range from about 5 to 90 % (v/v), usually from about 5 to 85 % (v/v), more usually from about 10 to 50 % (v/v) of the formulation.

Also present in the orally acceptable vehicle will be at least one non-ionic polyoxyalkylene surfactant, usually not more than two polyoxyalkylene non-ionic surfactants. The polyoxyalkylene surfactants will have a hydrophilic-lipophilic-balance (HLB) of from about 5 to 20, usually from about 8 to 16. Preferably, the polyoxyalkylene non-ionic surfactants employed in the subject formulations will be polyoxyethylene compounds. Polyoxyethylene compounds of interest include: ethoxylated alcohols, *i.e*. polyoxyethylene alcohols or ethoxylated fatty alcohols, where the alcohol moieties are generally of from 10 to 18, usually from 10 to 14 carbon atoms, as well as ether and ester substituents thereof; and polyoxyethylene derivatives of fatty acid partial esters, usually monoesters, of polyols of from 4 to 6 carbon atoms, usually 6 carbon atoms, where the polyols may be polyol anhydrides *e.g*. sorbitan. The fatty acid moieties of the subject surfactant will typically range from 10 to 18 carbon atoms. The number of ethylenoxide groups will generally be in the range of 2 to 30, usually in the range from about 2 to 25. Preferred surfactants are polyoxyethylene (4) lauryl ether (BRIJ 30®) and polyoxyethylene (20) mono sorbitan mono-oleate (TWEEN 80®). The total amount of non-ionic surfactants present in the subject formulations will range from 5 to 65 %, usually from about 5 to 60 % (v/v) of the formulation. Where TWEEN 80® is present in the formulation, it will usually be present in amounts ranging from 5 to 60 %, more usually from about 10 to 50 % (v/v) of the formulation. When BRIJ 30® is present in the subject formulation, it will usually be present in amounts ranging from 10 to 45 %, more usually from about 15 to 40 % (v/v) of the formulation.

The subject formulations may further comprise one or more cosolvents, usually not more than three different cosolvents, more usually not more than two different cosolvents, where suitable cosolvents include fatty acid esters and diols, where the cosolvent may be 100% fatty acid ester, 100% diol, or combination thereof. The total amount of cosolvent present in the formulation may range from about 20 to 80 % (v/v) and will usually range from about 25 to 75 % (v/v). When present in the formulation, the ratio of cosolvent to solvent in the subject formulations may range from about 1:1 to 15:1, but will usually range from about 1:1 to 13:1.

Fatty acid esters which may serve as cosolvents in the subject formulations are those fatty acid esters where the hydrocarbon chain of the fatty acid is from 12 to 18, usually 14 to 18 carbon atoms in length, where the fatty acid ester will be a mono-ester of a lower alkanol. Suitable fatty acid esters will generally comprise an even numbered fatty acid chain, where the hydrocarbon chain may be saturated or unsaturated, usually having not more than two sites of unsaturation. Fatty acids of interest will generally be of plant or mammalian origin and include palmitate, stearate, palmitoleate, linoleate, linolenate and the like, particularly myristate and oleate. The alcohol of the fatty acid mono-ester will be a lower alkanol of from 2 to 4 carbon atoms in length, usually 2 to 3 carbon atoms in length, with or without branches. Fatty acid esters of particular interest are isopropyl myristate and ethyl oleate. Isopropyl myristate, when present, will range from about 55 to 75 % (v/v), and ethyl oleate, when present, will range from about 35 to 75 % (v/v) of the total formulation. Usually the fatty acid ester will be present in an amount at least about equal (v/v) and up to 8 times the amount of surfactant in the formulation, usually not greater than 5 times the amount of surfactant in the formulation (v/v).

Diols may also be present in the subject formulations, where the diols may be present in addition to, or in lieu of, the fatty acid ester cosolvent. Diols of interest as cosolvents are generally liquids at physiologic temperatures and include diols of from 8 to 28 carbon atoms, usually 16 to 20 carbon atoms, where the diol may be a polyoxyalkylene diol, where alkylene is of from 2 to 3 carbon atoms. Suitable diols for use as cosolvents may range from about 200 to 800 daltons, usually from about 200 to 650 daltons. Diols of particular interest include polyethylene glycols, particularly polyethylene glycol 200 (PEG₂₀₀), polyethylene glycol 400 (PEG₄₀₀), polyethylene glycol 600 (PEG₆₀₀), and the like, with PEG₄₀₀ being preferred. When present as cosolvents in the subject formulations, the diols will range from about 5 to 60 % (v/v), usually from 5 to 55 % (v/v) of the formulation.

For formation of the amorphous nanoparticles, desirably in the formulation, the total amount of lower alkanol will generally be in the range of about 25-60 weight percent, more usually in the range of about 30-50 weight percent. The total amount of alkyleneoxy compound(s) will generally be in the range of about 20-50 weight percent, more usually in the range of about 25-40 weight percent. Where combinations of polyoxyalkylene compounds are employed, the amount of the fatty acid ester will generally range from about 25-100% of the polyoxyalkylene compounds.

In the subject formulations, the cosolvents themselves may impart desirable physical properties to the formulation, such as viscosity, stability and the like. Where desired, the formulation may further comprise additional agents which impart desired physical properties to the formulation, such as thickening agents, suspending agents, solidifying agents, and the like, where such agents include acacia, carboxymethylcellulose, hydroxypropylcellulose, lecithin, methyl cellulose, high molecular weight polyethylene glycols, e.g. those polyethylene glycols with molecular weights ranging from about 1000 to 6000, usually 1000 to 5000 daltons, povidone, sodium alginate, tragacanth, and the like . Also present in the subject formulations may be a number of minor components which provide various functions, such as enzyme inhibitors, preservatives, antioxidants, antimicrobial agents, stabilizers and the like. The total amount of these thickening agents and other additives, when present in the formulation, will normally not be greater than 5 weight %, usually 2 weight %, more usually 1 weight % of the formulation. A number of excipients may also be present in the subject formulations; as is known in the art.

The subject formulations are stable over a wide range of temperatures, where by stable is meant that the physical integrity of the formulation is not comprised, e.g. crystallization of the cyclosporin active agent does not occur. Included within the temperature range over which the subject formulations are stable are lower temperatures, such as those employed in refrigerated storage, where such lower temperatures typically range from about 0 to 15°C, more typically from about 2 to 8 °C.

The subject formulations are suitable for administration in capsule form, e.g. hard and soft capsules. Methods of producing hard capsules comprising liquid formulations are known in the art and described in U.S. Pat. Nos. 4,822,618 and 4,576,284. Generally, hard capsules that find use with the subject formulations will comprise two parts: a shell component and a cap component. The shell and cap components fit together to produce an enclosed cavity of defined volume sealed in a hard capsule shell. The shell and cap components may be fabricated from a hydrophilic polymer, such as starch or gelatin. In preparing the hard capsules, the liquid formulation will be poured into the shell component and then the capsule will be sealed by fitting the cap component over the shell component. The seal between the two components may be secured, thereby preventing leakage of the enclosed formulation from the capsule, by using a sealant as described in EP 116744. To avoid degradation in the stomach, capsules comprising the subject formulations may be coated with an enteric coating which inhibits degradation of the capsule in the acidic environment of the stomach. A variety of enteric coatings are known in the art. *See for example*, U.S. Pat. No. 5,206, 219.

The compositions, particularly the nanoparticle producing formulation, may be prepared by first dissolving the cyclosporin in the lower alkanol, where a small proportion of the polyoxyalkylene compound may also be included, generally less than about 50 weight percent of the composition used for dissolving the cyclosporin. An elevated temperature may be employed, usually in the range of about 60 to 90°C. After dissolving the cyclosporin, the major proportion of the polyalkyleneoxy compound may be added and the total formulation brought to the desired ratios by the addition of the appropriate components. Generally, the cyclosporin can be dissolved in the lower alkanol (optionally including a portion of the polyalkyleneoxy compound) at a weight ratio of about 1:1.5-5, more usually 1:2-4.

The subject formulations find use in immunosuppressive therapy. Immunosuppressive therapy is indicated in a wide variety of diseases, including idiopathic nephrotic syndrome, type I insulin-dependent diabetes, Behçet's syndrome, active Crohn's disease, aplastic anemia, severe corticosteroid-dependent asthma, psoriasis, rheumatoid arthritis, and other diseases where the immune system may play a pathogenic role. Of particular interest is the use of the subject formulations in transplant situations, including both allogeneic and xenogeneic organ, tissue or cell transplantation, where immunosuppression is desired to ensure maintained viability of the transplanted organ or tissue or cell following transplantation, *i.e*. to prevent graft rejection or prevent graft vs. host disease, *e.g.* following bone marrow transplantation.

In using the subject formulations to provide immunosuppressive therapy to a host, an effective amount of cyclosporin will be orally administered to achieve the desired level of immunosuppression in the host, depending on the particular condition to be treated. With transplantation, usually an initial dosage of cyclosporin will be administered prior to operation. Following transplantation of the donor organ to the host, the cyclosporin will be administered repeatedly, *i.e*. chronically, to the host to maintain immunosuppression. The initial dosage will be administered 4 to 12 hours prior to transplantation and may range from 10 to 18 mg/kg host, usually 10 to 15 mg/kg host. Following the operation, the initial dosage will usually be continued on a daily basis for a period of 1 to 3 weeks, usually 1 to 2 weeks. The dosage may then be tapered to a maintenance dosage of 3 to 10 mg/kg per day, usually 3 to 6 mg/kg per day. The rate at which the dosage is tapered to the maintenance level may range from 3 to 8 % per week and will usually be about 5 % per week. The dosage will typically be adjusted based on trough blood levels to maintain a concentration of 150 to 250 ng/ml, as measured by HPLC, RIA, ELISA or TDx assay. The subject formulations may be administered in conjunction with additional agents, where adjunct therapy is recommended and is known in the art. For example, the subject formulations may be administered in conjunction with adrenal corticosteroids, azathioprine and the like.

Administration of the subject formulations in conjunction with transplantation of a donor organ to a host will result in a prolongation of the viability of the donor organ in the host as a result of suppression of the host's immune response to the presence of the donor organ. By "prolongation of viability" is meant that the donor organ remains viable in the host for a longer period of time than it would have had immunosuppressive therapy not been employed in conjunction with the transplantation. Thus, prolongation of viability includes maintenance of viability for an indefinite period of time. A donor organ is considered viable as long as it maintains functionality in the host environment.

For convenience of the user, kits may be provided having the appropriate amount of cyclosporin, one or more dosage levels and the cosolvents, namely the lower alkanol(s) and the polyalkyleneoxy compound(s), e.g. at least one of ethanol and propylene glycol, and at least one of polysorbate 80 and PEG400.

The following examples are offered by way of illustration and not by way of limitation.

### EXPERIMENTAL

Several oral cyclosporin formulations according to the subject invention were prepared. The bioavailability of cyclosporin in the prepared formulations was then observed in rats and humans.

### I. Oral Cyclosporin Formulations

The following oral Cyclosporin A formulations were prepared. In each case, 100 mg CsA, the indicated amount of surfactant, and the indicated amount of ethanol or propylene glycol were added to a 1.0 ml volumetric flask, and the final volume of 1.0 ml was achieved by addition of a suitable volume of fatty acid ester and/or diol. cosolvent

| Formulation | Composition | | |
|---|---|---|---|
| 19 | EtOH | 0.1 ml | (10%) |
| | Tween 80 | 300 mg | (0.278 ml) |
| | IM | q.s. to 1.0 ml | ((0.622 ml) (531 mg) |
| 20 | EtOH | 0.05 ml | (5%) |
| | Brij 30 | 350 mg | (0.368 ml) |
| | IM | q.s. to 1.0 ml | ((0.582 ml)(496 mg) |
| 21 | PG | 0.05 ml | (5%) |
| | Brij 30 | 350 mg | (0.368 ml) |
| | IM | q.s. to 1.0 ml | ((0.582 ml)(496 mg) |
| 22 | EtOH | 0.1 ml | (10%) |
| | Tween 80 | 300 mg | (0.278 ml) |
| | EO | q.s. to 1.0 ml | <(0.622 ml)(541 mg) |
| 23 | EtOH | 0.05 ml | (5%) |
| | Brij 30 | 350 mg | (0.368 ml) |
| | EO | q.s. to 1.0 ml | <(0.582 ml) (506mg) |
| 24 | PG | 0.05 ml | (5%) |
| | Brij 30 | 350 mg | (0.368 ml) |
| | EO | q.s. to 1.0 ml | ((0.582 ml) (506mg) |
| 33 | EtOH | 0.1 ml | (10%) |
| | Brij 30 | 150 mg | (0.158 ml) |
| | IM | q.s. to 1.0 ml | ((0.742 ml)(633 mg) |
| 34 | EtOH | 0.1 ml | (10%) |
| | Brij 30 | 150 mg | (0.158 ml) |
| | EO | q.s. to 1.0 ml | ((0.742 ml)(646 mg) |
| 35 | EtOH | 0.1 ml | (10%) |
| | Tween 80 | 500 mg | (0.463 ml) |
| | PG | q.s. to 1.0 ml | ((0.437 ml)(453 mg) |
| 36 | EtOH | 0.1 ml | (10%) |
| | Tween 80 | 300 mg | (0.278 ml) |
| | PG | 100 mg | (0.097 ml) |
| | EO | q.s. to 1.0 ml | ((0.525 ml)(465 mg) |
| 37 | EtOH | 0.1 ml | (10 %) |
| | Tween 80 | 300 mg | (0.278 ml) |
| | PEG 400 | 100 mg | (0.088 ml) |
| | EO | q.s. to 1.0 ml | ((0.534 ml)(464 mg) |
| 38 | EtOH | 0.1 ml | (10%) |
| | Brij 30 | 300 mg | (0.316 ml) |
| | PG | 100 mg | (0.097 ml) |
| | EO | q.s. to 1.0 ml | ((0.487 ml)(424 mg) |
| 39 | EtOH | 0.1 ml | (10%) |
| | Brij 30 | 300 mg | (0.316 ml) |
| | PG | 200 mg | (0.193 ml) |
| | EO | q.s. to 1.0 ml | ((0.391 ml)(340 mg) |
| 40 | PG | 300 mg | (290 ml) |
| | Brij 30 | 300 mg | (0.316 ml) |
| | EO | q.s. to 1.0 ml | ((0.394 ml)(343 mg) |
| 41 | EtOH | 0.05 ml | (5%) |
| | Brij 30 | 150 mg | (0.158 ml) |
| | Tween 80 | 100 mg | (0.093 ml) |
| | EO | q.s. to 1.0. ml | ((0.649 ml) (565 mg) |
| 42 | PG | 0.05 ml | (5%) |
| | Brij 30 | 150mg | (0.158 ml) |
| | Tween 80 | 100 mg | (0.093 ml) |
| | EO | q.s. to 1.0. ml | ((0.649 ml) (565 mg) |
| 43 | EtOH | 0.10 ml | (10%) |
| | Tween 80 | 400 mg | (0.371 ml) |
| | PG | q.s. to 1.0 ml | (0.529 ml) |
| 44 | EtOH | 0.10 ml | (10%) |
| | Tween 80 | 400 mg | (0.371 ml) |
| | PEG₄₀₀ | q.s. to 100 ml | ((0.529 ml)(601 mg) |
| 45 | EtOH | 0.10 ml | |
| | Tween 80 | 300 mg | (0.278 ml) |
| | PG | approx.250mg | (0.243 ml) |
| | PEG₄₀₀ | approx.250mg | (0.220 ml) |
| 46 | EtOH | 0.10 ml | (10%) |
| | Tween 80 | 100 mg | (0.093 ml) |
| | PG | q.s. to 1.0 ml | (0.807 ml) |
| 48 | EtOH | 0.10 ml | |
| | Tween 80 | 200 mg | (0.186 ml) |
| | PG | approx.250mg | (0.243 ml) |
| | PEG₄₀₀ | approx.250mg | (0.220 ml) |
| 49 | EtOH | 0.10 ml | (10%) |
| | Tween 80 | 600 mg | (0.558 ml) |
| | PG | q.s. to 1 ml | (0.342 ml) |
| 50 | EtOH | 0.10 ml | (10%) |
| | Tween 80 | 300 mg | (0.278 ml) |
| | PG | q.s. to 1.0 ml | (0.622 ml) |
| 51 | EtOH | 0.10 ml | (10 %) |
| | Tween 80 | 200 mg | (0.186 ml) |
| | PG | q.s. to 1.0 ml | (0.714 ml) |
| 52 | EtOH | 0.05 ml | (5%) |
| | Tween 80 | 400 mg | (0.371 ml) |
| | PG | q.s. to 1.0 ml | (0.579 ml) |
| PG = Propylene Glycol; EtOH = ethanol | | | |
| Brij 30 = polyoxyethylene (4) lauryl ether | | | |
| Tween 80 = polyoxyethylene (20) mono sorbitan mono-oleate | | | |
| IM=isopropyl myristate | | | |
| EO=ethyl oleate | | | |

### II. In vivo Bioavailability Studies for Formulations 19-24 and 33-42

The bioavailability of cyclosporin in formulations 19-24 and 33-42 was studied as follows. As a measure of bioavailability, the following pharmacokinetic parameters were determined: (a) the peak blood concentration of cyclosporin (Cₘₐₓ); (b) time required to attain Cₘₐₓ (Tₘₐₓ); and the area under the blood concentration time-curve time (AUC). In addition to formulations 19-24 and 33-42, the bioavailability of cyclosporin in SANDIMMUNE® Oral Solution (SO) under analogous conditions was observed for comparison purposes. For each of the above formulations, CsA-naive Sprague Dawley rats weighing 250-350 gm were fed pelletized standard food (Agway® 3000, Granville Mill, Greensboro, NC) and water *ad libitum.* One day prior to the experiment, silicone rubber cannulae were inserted into the right jugular and right femoral veins under light ether anesthesia. After overnight fast, CsA was administered by gavage.

Following administration, 200µl blood samples were collected from the jugular vein in 0.5 ml polypropylene microfuge tubes containing 0.3 mg of lyophilized Na EDTA and vortexed immediately for 10 sec. The sampling times for animals subjected to oral formulations were 0, 0.5, 1, 2, 4, 8, 12, 24, 36, 48 and 72 hr after administration.

CsA, including some of its metabolites, was determined in whole blood by fluorescent polarization immunoassay (FPI)(TDx, Abbot Lab.). Briefly, 150 µl of the whole blood sample were quantitatively transferred to a 1.5 ml microfuge tube. Cells were lysed and dissolved with 50 µl of a surfactant-containing solubilizing reagent. Proteins were then precipitated out with 300 µl of acetonitrile. After centrifugation, the supernatant was subjected to the FPI assay in a TDx Autoanalyzer following the procedure recommended by Abbott Diagnostics. Since the TDx assay was originally developed for human blood, some of the recommended procedures were modified as follows. A series of standard solutions of known CsA concentration were prepared by adding a known amount of CsA to rat blood treated with EDTA. When the CsA concentration in a sample was expected to be greater than 1.0 µg/ml, the blood sample was diluted 10-fold in a 0.1 M-phosphate buffer at pH.7.0. For diluted samples, another calibration curve was made using a series of standard solutions containing known amounts of CsA, which is volume-wise 10% in rat blood and 90% phosphate buffer:

Descriptive pharmacokinetic parameters were obtained from non-compartmental analyses. The peak concentration (Cₘₐₓ) and the time at which the peak concentration occurred (Tₘₐₓ) were estimated by inspection of the raw concentration-time profile for each rat. The area under the blood concentration-time curve (AUC) from time 0 through the last data point (AUC₀₋ₜ) was calculated according to the linear trapezoidal procedure. The residual area under the tail of the blood concentration-time curve (AUC_{t-∞}) was estimated as the ratio of the final observed concentration (C*) to the first-order rate constant associated with the terminal elimination phase of the concentration-time profile (λ_{z}). The rate contact λ_{z} was determined by log-linear regression of the conceritration-time data in the apparent terminal log-linear phase of the concentration-time profile (i.e., the final 3 to 5 data points, depending on the profile under analysis). The total AUC (AUC_{t-∞}) was taken as the sum of AUC₀₋ₜ and AUC_{t-∞}.

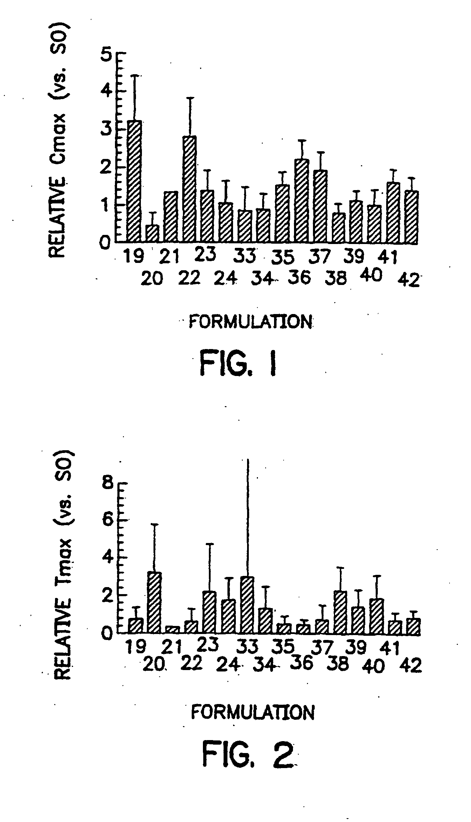

The results for each formulation were compared with the results obtained for SO, and are provided in Figs. 1-3. The results demonstrate that, for the majority of the formulations, greater bioavailability of cyclosporin is achieved with the subject formulations as compared with SANDIMMUNE® Oral Solution (SO), as indicated by the higher AUC values of the subject formulations.

### III. In vivo Human Bioavailability of Formulations 35, 43-46 and 48-52.

48 healthy males between the ages of 19 and 55 with no more than 20 % deviation from ideal weight were used as test subjects. A single dose, fasted, randomized, double-blinded, three-way crossover study was conducted. The 48 subjects were randomized into 6 groups of 8 subjects. Each group received a single 300 mg dose of cyclosporin from the above formulations, or SANDIMMUNE® Oral Solution (SO), on three different occasions, where each occasion was separated by a 7-day washout period.

Subjects were required to fast 10 hours prior to, and 4 hours after, dosing. Water was allowed ad lib during the study, except for a 1 hour period prior through 2 hours following dosing. Prior to dosing, a 15 ml blood sample was drawn. For administrations, 3 ml aliquots of formulation (300 mg) was combined with 200 ml chocolate milk and orally ingested. 10 ml blood samples were drawn at t= 0, 0.5, 1, 1.5, 2, 3, 4, 6, 8, 10, 12, 16, 20 and 24 hours. A post study 15 ml blood sample was also drawn.

Concentrations of cyclosporin A in the whole blood samples were assayed using the TDx (Abbott Diagnostics, N. Chicago, IL) according to the manufacturer's instructions.

Non-compartmental pharmacokinetics were derived using standard methods. The maximum whole blood concentration (Cₘₐₓ) and the time of its occurrence (Tₘₐₓ) were compiled from the concentration-time data. The area under the blood concentration time curve (AUC) was calculated by the linear trapezoidal rule to the last blood concentration above the limit of sensitivity (25 ng/ml) and extrapolated to infinity.

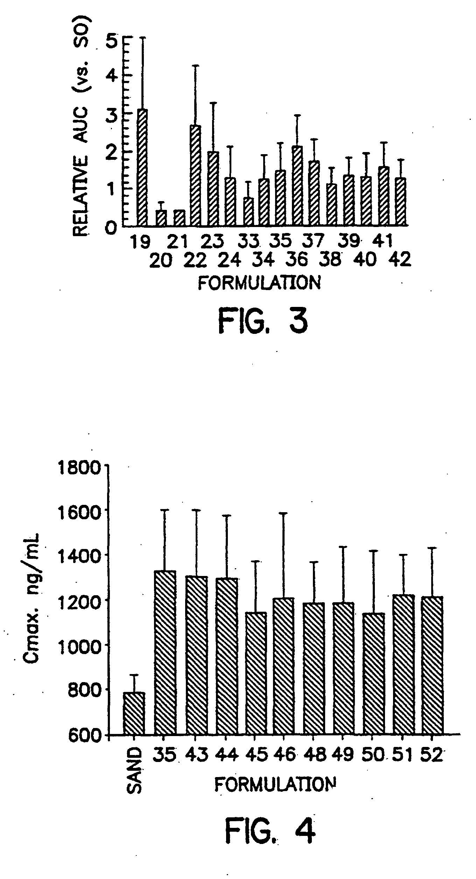

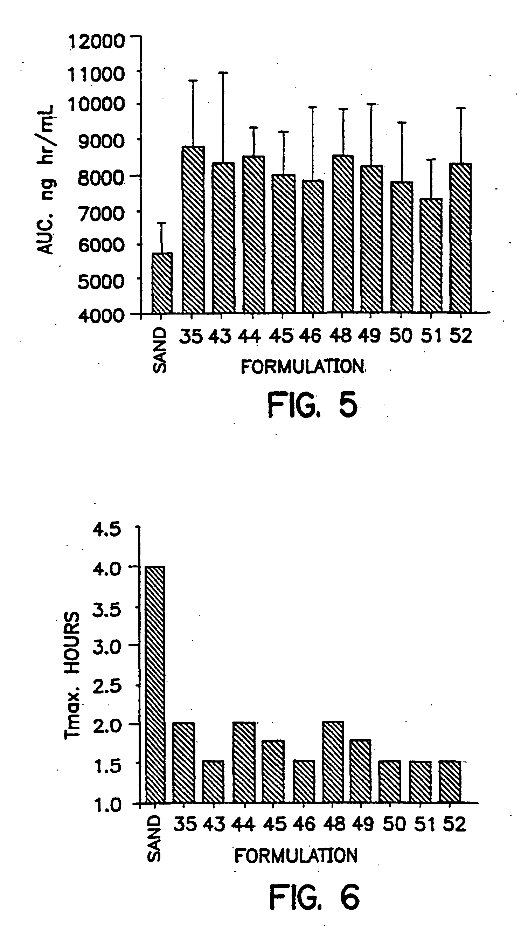

The observed Cₘₐₓ, Tₘₐₓ and AUC values for each formulation were averaged. The average values for each formulation are provided in Figures 4-6. The results demonstrate that for each formulation tested, Cmax occurred at least twice as fast as with SANDIMMUNE® Oral Solution (SO) under the same conditions. Furthermore, the AUC observed for the test formulations was at least 2000 ng·hr/ml greater than that observed for SANDIMMUNE® Oral Solution (SO) under the same conditions. Based on these results, formulations 35, 43-46 and 48-52 provide for greater bioavailability than SANDIMMUNE® Oral Solution (SO).

Formulations were prepared for the formationof amorphous nanoparticles on dilution in an aqueous medium.

### IV. Nanoparticle Formulations

A. 5 g of cyclosporin A was added to 5 mL of ethanol. The mixture was stirred to complete dissolution of cyclosporin A. To the resulting solution were added 25 g of polysorbate 80 and the volume is completed to 50 mL by 1,2-propylene glycol. The mixture was sufficiently stirred at room temperature until a homogeneous solution was formed.
B. 5 g of cyclosporin A was added to 5 mL of ethanol. The mixture was stirred until complete dissolution of cyclosporin A. To the resulting solution were added 15 g of polysorbate 80 and the volume is completed to 50 mL by a mixture of 1,2-propylene glycol and polyethylene glycol 400. The mixture was sufficiently stirred at room temperature until a homogeneous solution was formed.
C. 1 mL of the solution obtained in example 1 was added in 50 mL of water with a glass syringe as recommended for the oral administration of concentrated emulsions or microemulsions in human. The addition of the solution was followed by a quick dissolution and a white suspension of fine particles was obtained having a blue reflect as colloidal suspensions (Tyndall effect). After centrifugation at 26,000 g during 5 hours, the sediment was washed with water and then centrifuged at 26,000 g during 24 hours. The washing and centrifugation processes were repeated twice under the same conditions. After drying, an x-ray powder diagram was performed. The solid was exclusively in amorphous form.
   The sediment was examined by scanning electron microscopy. The sediment was constituted of amorphous spheric nanoparticles with a diameter between 200 and 400 nm with the presence of some aggregates.
D. 2 mL of the solution obtained in example 1 was added in 100 mL of water and the colloidal suspension was examined 10 minutes and 1 hour after the dilution by a diffraction/diffusion laser granulometer (Malvern SB.OD).
   After 1 hour, two particle populations were observed: one representing 70% of the weight of cyclosporin A with an average diameter of 300 nm and a second one representing 30% of the weight of cyclosporin A with an average diameter of 20 µm, probably constituting aggregates of nanoparticles.
E. 1 mL of the solution obtained in example 1 was added to 50 mL of water and the colloidal suspension was stirred during 10 minutes.
   The suspension was then added to 200 mL of artificial acidic gastric juice and warmed at 37°C. The homogeneous colloidal suspension was examined by diffraction/diffusion laser granulometry (Malvern SB.OD). The suspension was constituted exclusively of nanoparticles with an average diameter of 600 nm.
F. 1 mL of the solution obtained in example 1 was added directly to 200 mL of artificial acidic gastric juice.

The homogeneous suspension was warmed at 37°C and examined rapidly by diffraction/diffusion laser granulometry (Malvern SB.OD). The suspension was exclusively constituted of nanoparticles with an average diameter of 350 nm.

From the above results and discussion, it is evident that novel cyclosporin formulations having high bioavailability are provided. The subject formulations are capable of comprising high concentrations of cyclosporin and are storage stable over a wide range of temperatures, including low temperatures commonly used in refrigeration. The subject formulations are amenable to delivery in capsule form, including hard capsule form, providing for ease of storage and handling. The formulations also provide amorphous nanoparticles, which result in enhancced bioavailability of the cyclosporin.

## Claims

1. An oral cyclosporin formulation consisting essentially of:
cyclosporin;
at least one alkanol solvent of from 2 to 3 carbon atoms;
at least one non-ionic polyoxyalkylene surfactant selected from the group consisting of polyoxyethylene alcohols and fatty acid monoesters of ethoxylated polyols of from 4 to 6 carbons atoms; and optionally
at least one cosolvent selected from the group consisting of monoesters of a lower alkanol of from 2 to 4 carbon atoms and a fatty acid of from 14 to 18 carbon atoms; and diols of from 8 to 28 carbon atoms,
where the diols may be polyoxyalkylene diols, where alkylene is from 2 to 3 carbon atoms.

2. The formulation as claimed in claim 1 wherein said at least one alkanol solvent is selected from the group comprising ethanol and propylene glycol.

3. The formulation as claimed in claim 1 or claim 2 wherein the formulation comprises ethanol and propylene glycol.

4. The formulation as claimed in any one of claims 1 to 3 wherein ethanol is present in an amount of from 5 to 20% (v/v) of said formulation.

5. The formulation as claimed in any preceding claim wherein propylene glycol is present in an amount of from 10 to 50% (v/v) of said formulation.

6. The formulation as claimed in any preceding claim wherein the total of alkanol solvent in the formulation is from 5 to 75% (v/v) of said formulation.

7. The formulation as claimed in any preceding claim wherein said fatty acids monoesters of ethoxylated polyols of from 4 to 6 carbon atoms comprise monoesters of polyol anhydrides.

8. The formulation as claimed in any preceding claim wherein said ethoxylated polyols of from 4 to 6 carbon atoms comprise sorbitan.

9. The formulation as claimed in any one of claims 1 to 6 wherein said at least one non-ionic polyoxyalkylene surfactant comprises polyoxyethylene (4) lauryl ether.

10. The formulation as claimed in any preceding claim wherein said at least one non-ionic polyoxyalkylene surfactant comprises polyoxyethylene (20) monosorbitan monooleate.

11. The formulation as claimed in any preceding claim wherein said at least one non-ionic polyoxyalkylene surfactant is from 5 to 65% (v/v) of said formulation.

12. The formulation as claimed in any preceding claim comprising said at least one cosolvent selected from the group consisting of monoesters of a lower alkanol of from 2 to 4 carbon atoms and a fatty acid of from 14 to 18 carbon atoms; and diols of from 8 to 28 carbon atoms, where the diols may be polyoxyalkylene diols, where alkylene is from 2 to 3 carbon atoms.

13. The formulation as claimed in claim 12 wherein said monoesters are selected from the group comprising isopropyl myristate and ethyl oleate.

14. The formulation as claimed in either claim 12 or claim 13 wherein said cosolvent comprises a polyethylene glycol.

15. The formulation as claimed in claim 14 wherein said polyethylene glycol is selected from the group comprising polyethylene glycol 200, polyethylene glycol 400 and polyethylene glycol 800.

16. The formulation as claimed in any one of claims 12 to 15 wherein said at least one cosolvent is from 20 to 80% (v/v) of said formulation.

17. The formulation as claimed in any preceding claim comprising two cosolvents.

18. The formulation as claimed in any preceding claim wherein said cyclosporin is Cyclosporin A.

19. The formulation as claimed in claim 18 wherein the Cyclosporin A is at a concentration ranging from 50 to 150 mg/ml.

20. A hard capsule for oral administration of cyclosporin, said capsule comprising a shell component and a cap component enclosing a cyclosporin formulation comprising:
cyclosporin;
at least one alkanol solvent of from 2 to 3 carbon atoms;
at least one non-ionic polyoxyalkylene surfactant; and
a polyethylene glycol cosolvent,
**characterised in that** said liquid cyclosporin formulation produces amorphous bioavailable cyclosporin nanoparticles upon dilution into an aqueous medium.

21. The hard capsule as claimed in claim 20, wherein said shell component is fabricated from a hydrophilic polymer.

22. A kit consisting essentially of:
a cyclosporin;
at least one alkanol solvent of from 2 to 3 carbon atoms;
at least one non-ionic polyoxyalkylene surfactant, wherein said surfactant is selected from the group consisting of polyoxyethylene alcohols and fatty acid monoesters of ethoxylated polyols of from 4 to 6 carbon atoms; and optionally
at least one cosolvent selected from the group consisting of monoesters of a lower alkanol of from 2 to 4 carbon atoms and a fatty acid of from 14 to 18 carbon atoms; and diols of from 8 to 28 carbon atoms,
where the diols may be polyoxyalkylene diols, where alkylene is from 2 to 3 carbon atoms.

23. A kit as claimed in claim 22 comprising at least one of ethanol and propylene glycol, and at least one of polysorbate 80 and PEG 400.

24. A method for preparing an oral cyclosporin formulation comprising:
cyclosporin;
at least one alkanol solvent of from 2 to 3 carbon atoms; and
at least one non-ionic polyoxyalkylene surfactant,
said method comprising the steps of:
(i) dissolving cyclosporin in at least one alkanol solvent of from 2 to 3 carbon atoms; and
(ii) adding at least one non-ionic polyoxyalkylene surfactant,
**characterised in that** said formulation produces amorphous bioavailable cyclosporin nanoparticles upon dilution into an aqueous medium.

25. The method as claimed in claim 24 wherein step (ii) further comprises, after addition of said at least one non-ionic polyoxyalkylene surfactant, the addition of at least one cosolvent selected from the group consisting of monoesters of a lower alkanol of from 2 to 4 carbon atoms and a fatty acid of from 14 to 18 carbon atoms; and diols of from 8 to 28 carbon atoms, where the diols may be polyoxyalkylene diols, where alkylene is from 2 to 3 carbon atoms, preferably a polyethylene glycol cosolvent.

26. The method as claimed in claim 24 wherein said formulation is as defined in any one of claims 1 to 19.

27. The method as claimed in any one of claims 24 to 26 in which an amount of said at least one non-ionic polyoxyalkylene surfactant is included in the said at least one alkanol solvent in which the cyclosporin is dissolved, said amount being up to 50 weight percent of the composition used for dissolving the cyclosporin.

28. A method of forming an aqueous dispersion of cyclosporin particles comprising the step of diluting in an aqueous medium the formulation produced by the method of any one of claims 24 to claim 27.

29. The method as claimed in claim 28 wherein said aqueous medium is water.

30. The method of claim 28 or claim 29, wherein at least 50 weight percent of the cyclosporin present in said aqueous dispersion is of particles less than about 1 µm, said cyclosporin being amorphous.

31. Use of an aqueous dispersion of cyclosporin particles prepared by a method according to any one of claims 28 to 30 in the manufacture of a medicament for treating a patient in need of immunosuppressive treatment.

## Patentansprüche

1. Orale Cyclosporinformulierung bestehend im wesentlichen aus:
Cyclosporin;
wenigstens einem alkanolischen Lösungsmittel mit 2 bis 3 Kohlenstoffatomen;
wenigstens einem nichtionischen oberflächenaktiven Polyoxyalkylen ausgewählt aus der Gruppe bestehend aus Polyoxyethylenalkoholen und Fettsäuremonoestern ethoxylierter Polyole mit 4 bis 6 Kohlenstoffatomen; und optional
wenigstens einem Kolösungsmittel ausgewählt aus der Gruppe bestehend aus Monoestern eines niederen Alkanols mit 2 bis 4 Kohlenstoffatomen und einer Fettsäure mit 14 bis 18 Kohlenstoffatomen, und Diolen mit 8 bis 28 Kohlenstoffatomen, wobei die Diole Polyoxyalkylendiole sein können und wobei das Alkylen 2 bis 3 Kohlenstoffatome hat.

2. Formulierung gemäß Anspruch 1, wobei das wenigstens eine alkanolische Lösungsmittel aus der Gruppe umfassend Ethanol und Propylenglykol ausgewählt ist.

3. Formulierung gemäß Anspruch 1 oder 2, wobei die Formulierung Ethanol und Propylenglykol umfaßt.

4. Formulierung gemäß einem der Ansprüche 1 bis 3, wobei Ethanol in einer Menge von 5 bis 20% (V/V) der Formulierung vorhanden ist.

5. Formulierung gemäß einem der vorhergehenden Ansprüche, wobei Propylenglykol in einer Menge von 10 bis 50% (V/V) der Formulierung vorhanden ist.

6. Formulierung gemäß einem der vorhergehenden Ansprüche, wobei die Gesamtmenge des alkanolischen Lösungsmittels in der Formulierung 5 bis 75% (V/V) der Formulierung beträgt.

7. Formulierung gemäß einem der vorhergehenden Ansprüche, wobei die Fettsäuremonoester der ethoxylierten Polyole mit 4 bis 6 Kohlenstoffatomen Monoester von Polyolanhydriden umfassen.

8. Formulierung gemäß einem der vorhergehenden Ansprüche, wobei die ethoxylierten Polyole mit 4 bis 6 Kohlenstoffatomen Sorbitan umfassen.

9. Formulierung gemäß einem der Ansprüche 1 bis 6, wobei das wenigstens eine nichtionische oberflächenaktive Polyoxyalkylen Polyoxyethylen-(4)-laurylether umfaßt.

10. Formulierung gemäß einem der vorhergehenden Ansprüche, wobei das wenigstens eine nichtionische oberflächenaktive Polyoxyalkylen Polyoxyethylen-(20)-monosorbitanmonooleat umfaßt.

11. Formulierung gemäß einem der vorhergehenden Ansprüche, wobei das wenigstens eine nichtionische oberflächenaktive Polyoxyalkylen von 5 bis 65% (V/V) der Formulierung beträgt.

12. Formulierung gemäß einem der vorhergehenden Ansprüche, umfassend das wenigstens eine Kolösungsmittel ausgewählt aus der Gruppe bestehend aus Monoestern eines niederen Alkanols mit 2 bis 4 Kohlenstoffatomen und einer Fettsäure mit 14 bis 18 Kohlenstoffatomen, und Diolen mit 8 bis 28 Kohlenstoffatomen, wobei die Diole Polyoxyalkylendiole sein können und wobei das Alkylen 2 bis 3 Kohlenstoffatome hat.

13. Formulierung gemäß Anspruch 12, wobei die Monoester aus der Gruppe umfassend Isopropylmyristat und Ethyloleat ausgewählt sind.

14. Formulierung gemäß 12 oder Anspruch 13, wobei das Kolösungsmittel ein Polyethylenglykol umfaßt.

15. Formulierung gemäß Anspruch 14, wobei das Polyethylenglykol aus der Gruppe umfassend Polyethylenglykol 200, Polyethylenglykol 400 und Polyethylenglykol 800 ausgewählt ist.

16. Formulierung gemäß einem der Ansprüche 12 bis 15, wobei das wenigstens eine Kolösungsmittel von 20 bis 80% (V/V) der Formulierung beträgt.

17. Formulierung gemäß einem der vorhergehenden Ansprüche umfassend zwei Kolösungsmittel.

18. Formulierung gemäß einem der vorhergehenden Ansprüche, wobei das Cyclosporin Cyclosporin A ist.

19. Formulierung gemäß Anspruch 18, wobei das Cyclosporin A eine Konzentration im Bereich von 50 bis 150 mg/ml hat.

20. Feste Kapsel zur oralen Verabreichung von Cyclosporin, umfassend eine Hüllenkomponente und eine Aufsatzkomponente umschließend eine Cyclosporinformulierung umfassend
Cyclosporin;
wenigstens ein alkanolisches Lösungsmittel mit 2 bis 3 Kohlenstoffatomen;
wenigstens ein nichtionisches oberflächenaktives Polyoxyalkylen, und
ein Polyethylenglykol-Kolösungsmittel,
**dadurch gekennzeichnet, dass** die flüssige Cyclosporinformulierung amorphe bioverfügbare Cyclosporin-Nanopartikel nach Verdünnen in einem wässerigen Medium ergibt.

21. Feste Kapsel gemäß Anspruch 20, wobei die Hüllenkomponente aus einem hydrophilen Polymer hergestellt ist.

22. Kit, bestehend im wesentlichen aus:
einem Cyclosporin;
wenigstens einem alkanolischen Lösungsmittel mit 2 bis 3 Kohlenstoffatomen;
wenigstens einem nichtionischen oberflächenaktiven Polyoxyalkylen ausgewählt aus der Gruppe bestehend aus Polyoxyethylenalkoholen und Fettsäuremonoestem ethoxylierter Polyole mit 4 bis 6 Kohlenstoffatomen; und optional
wenigstens einem Kolösungsmittel ausgewählt aus der Gruppe bestehend aus Monoestem eines niederen Alkanols mit 2 bis 4 Kohlenstoffatomen und einer Fettsäure mit 14 bis 18 Kohlenstoffatomen, und Diolen mit 8 bis 28 Kohlenstoffatomen, wobei die Diole Polyoxyalkylendiole sein können und wobei das Alkylen 2 bis 3 Kohlenstoffatome hat.

23. Kit gemäß Anspruch 22 umfassend wenigstens eines von Ethanol und Propylenglykol und wenigstens eines von Polysorbat 80 und PEG 400.

24. Verfahren zur Herstellung einer oralen Cyclosporinformulierung umfassend:
Cyclosporin;
wenigstens ein alkanolisches Lösungsmittel mit 2 bis 3 Kohlenstoffatomen; und
wenigstens ein nichtionisches oberflächenaktives Polyoxyalkylen,
wobei das Verfahren die Schritte umfasst:
(i) Lösen von Cyclosporin in wenigstens einem alkanolischen Lösungsmittel mit 2 bis 3 Kohlenstoffatomen; und
(ii) Hinzufügen von wenigstens einem nichtionischen oberflächenaktiven Polyoxyalkylen,
**dadurch gekennzeichnet, dass** die Formulierung amorphe bioverfügbare Cyclosporin-Nanopartikel nach Verdünnen in einem wässerigen Medium ergibt.

25. Verfahren gemäß Anspruch 24, wobei Schritt (ii) weiterhin das Hinzufügen von wenigstens einem Kolösungsmittel nach Hinzufügen des wenigstens einen nichtionischen oberflächenaktiven Polyoxyalkylens umfasst, wobei das wenigstens eine Kolösungsmittel aus der Gruppe bestehend aus Monoestern eines niederen Alkanols mit 2 bis 4 Kohlenstoffatomen und einer Fettsäure mit 14 bis 18 Kohlenstoffatomen, und Diolen mit 8 bis 28 Kohlenstoffatomen ausgewählt wird, wobei die Diole Polyoxyalkylendiole sein können und wobei das Alkylen 2 bis 3 Kohlenstoffatome hat, vorzugsweise von einem Polyethylenglykol-Kolösungsmittel.

26. Verfahren gemäß Anspruch 24, wobei die Formulierung gemäß einem der Ansprüche 1 bis 19 definiert ist.

27. Verfahren gemäß einem der Ansprüche 24 bis 26, in welchem die Menge des wenigstens einen nichtionischen oberflächenaktiven Polyoxyalkylens in das wenigstens eine alkanolische Lösungsmittel eingeschlossen wird, in dem das Cyclosporin gelöst wird, wobei die Menge bis zu 50 Gew.-% der Zusammensetzung beträgt, die zum Lösen des Cyclosporins verwendet wird.

28. Verfahren zur Bildung einer wässerigen Dispersion von Cyclosporinpartikeln, umfassend den Schritt des Verdünnens der Formulierung, die durch das Verfahren gemäß einem der Ansprüche 24 bis 27 hergestellt wird, in einem wässerigen Medium.

29. Verfahren gemäß Anspruch 28, wobei das wässerige Medium Wasser ist.

30. Verfahren gemäß Anspruch 28 oder Anspruch 29, wobei wenigstens 50 Gew.-% des Cyclosporins, das in der wässerigen Dispersion vorhanden ist, aus Partikeln kleiner als ungefähr 1 µm besteht und wobei das Cyclosporin amorph ist.

31. Verwendung einer wässerigen Dispersion von Cyclosporinpartikeln, die durch das Verfahren gemäß einem der Ansprüche 28 bis 30 hergestellt wird, zur Herstellung eines Medikamentes zur Behandlung eines Patienten, der eine immunsuppressive Behandlung benötigt.

## Revendications

1. Formulation de cyclosporine par voie orale composée essentiellement de :
- cyclosporine ;
- au moins un solvant alcanol ayant 2 à 3 atomes de carbone ;
- au moins un tensio-actif polyoxyalkylène non ionique choisi dans le groupe formé par les polyoxyéthylène-alcools et les monoesters d'acide gras et de polyols éthoxylés ayant 4 à 6 atomes de carbone ; et éventuellement
- au moins un co-solvant choisi dans le groupe formé par les monoesters d'un alcanol inférieur ayant 2 à 4 atomes de carbone et d'un acide gras ayant 14 à 18 atomes de carbone ; et les diols ayant 8 à 28 atomes de carbone, où les diols peuvent être des polyoxyalkylène-diols, où l'alkylène possède 2 à 3 atomes de carbone.

2. Formulation selon la revendication 1, dans laquelle ledit au moins un solvant alcanol est choisi dans le groupe comprenant l'éthanol et le propylèneglycol.

3. Formulation selon la revendication 1 ou 2, dans laquelle la formulation comprend l'éthanol et le propylèneglycol.

4. Formulation selon l'une quelconque des revendications 1 à 3, dans laquelle l'éthanol est présent à raison de 5 à 20% (v/v) de ladite formulation.

5. Formulation selon l'une quelconque des revendications précédentes, dans laquelle le propylèneglycol est présent à raison de 10 à 50% (v/v) de ladite formulation.

6. Formulation selon l'une quelconque des revendications précédentes, dans laquelle la totalité de solvant alcanol dans la formulation représente 5 à 75% (v/v) de ladite formulation.

7. Formulation selon l'une quelconque des revendications précédentes, dans laquelle lesdits monoesters d'acides gras et de polyols éthoxylés ayant 4 à 6 atomes de carbone comprennent les monoesters de polyol-anhydrides.

8. Formulation selon l'une quelconque des revendications précédentes, dans laquelle lesdits polyols éthoxylés ayant 4 à 6 atomes de carbone comprennent le sorbitan.

9. Formulation selon l'une quelconque des revendications 1 à 6, dans laquelle ledit au moins un tensio-actif polyoxyalkylène non ionique comprend l'éther polyoxyéthylène(4)-laurylique.

10. Formulation selon l'une quelconque des revendications précédentes, dans laquelle ledit au moins un tensio-actif polyoxyalkylène non ionique comprend le monooléate de polyoxyéthylène(20)-monosorbitan.

11. Formulation selon l'une quelconque des revendications précédentes, dans laquelle ledit au moins un tensio-actif polyoxyalkylène non ionique représente 5 à 65% (v/v) de ladite formulation.

12. Formulation selon l'une quelconque des revendications précédentes, comprenant ledit au moins un co-solvant choisi dans le groupe formé par les monoesters d'un alcanol inférieur ayant 2 à 4 atomes de carbone et d'un acide gras ayant 14 à 18 atomes de carbone ; et les diols ayant 8 à 28 atomes de carbone, où les diols peuvent être des polyoxyalkylène-diols, où l'alkylène possède 2 à 3 atomes de carbone.

13. Formulation selon la revendication 12, dans laquelle lesdits monoesters sont choisis dans le groupe comprenant le myristate d'isopropyle et l'oléate d'éthyle.

14. Formulation selon la revendication 12 ou 13, dans laquelle ledit co-solvant comprend un polyéthylèneglycol.

15. Formulation selon la revendication 14, dans laquelle ledit polyéthylèneglycol est choisi dans le groupe comprenant le polyéthylèneglycol 200, le polyéthylèneglycol 400 et le polyéthylèneglycol 800.

16. Formulation selon l'une quelconque des revendications 12 à 15, dans laquelle ledit au moins un co-solvant représente 20 à 80% (v/v) de ladite formulation.

17. Formulation selon l'une quelconque des revendications précédentes, comprenant deux co-solvants.

18. Formulation selon l'une quelconque des revendications précédentes, dans laquelle ladite cyclosporine est la cyclosporine A.

19. Formulation selon la revendication 18, dans laquelle la cyclosporine A est à une concentration allant de 50 à 150 mg/mL.

20. Gélule pour une administration par voie orale de cyclosporine, ladite gélule comprenant un composant de de coquille et un composant de coiffe enfermant une formulation de cyclosporine, comprenant :
- cyclosporine ;
- au moins un solvant alcanol ayant 2 à 3 atomes de carbone ;
- au moins un tensio-actif polyoxyalkylène non ionique ; et
- un co-solvant de polyéthylèneglycol ;
**caractérisée en ce que** ladite formulation de cyclosporine liquide produit des nanoparticules de cyclosporine biodisponibles amorphes après dilution dans un milieu aqueux.

21. Gélule selon la revendication 20, dans laquelle ledit composant de coquille est fabriqué à partir d'un polymère hydrophile.

22. Trousse composée essentiellement de :
- cyclosporine ;
- au moins un solvant alcanol ayant 2 à 3 atomes de carbone ;
- au moins un tensio-actif polyoxyalkylène non ionique, dans lequel ledit tensio-actif est choisi dans le groupe formé par les polyoxyéthylène-alcools et les monoesters d'acide gras et de polyols éthoxylés ayant 4 à 6 atomes de carbone ; et éventuellement
- au moins un co-solvant choisi dans le groupe formé par les monoesters d'un alcanol inférieur ayant 2 à 4 atomes de carbone et d'un acide gras ayant 14 à 18 atomes de carbone ; et les diols ayant 8 à 28 atomes de carbone, où les diols peuvent être des polyoxyalkylène-diols, où l'alkylène possède 2 à 3 atomes de carbone.

23. Trousse selon la revendication 22, comprenant au moins un élément parmi l'éthanol et le propylèneglycol, et au moins un élément parmi le polysorbate 80 et le PEG 400.

24. Procédé pour préparer une formulation de cyclosporine par voie orale comprenant :
- une cyclosporine ;
- au moins un solvant alcanol ayant 2 à 3 atomes de carbone ; et
- au moins un tensio-actif polyoxyalkylène non ionique ;
ledit procédé comprenant les étapes consistant à :
(i) dissoudre la cyclosporine dans au moins un solvant alcanol ayant 2 à 3 atomes de carbone ; et
(ii) ajouter au moins un tensio-actif polyoxyalkylène non ionique,
**caractérisé en ce que** ladite formulation produit des nanoparticules de cyclosporine biodisponibles amorphes après dilution dans un milieu aqueux.

25. Procédé selon la revendication 24, dans lequel l'étape (ii) comprend de plus, après addition dudit au moins un tensio-actif polyoxyalkylène non ionique, l'addition d'au moins un co-solvant choisi dans le groupe formé des monoesters d'un alcanol inférieur ayant 2 à 4 atomes de carbone et d'un acide gras ayant 14 à 18 atomes de carbone ; et les diols ayant 8 à 28 atomes de carbone, où les diols peuvent être les polyoxyalkylène-diols, où l'alkylène possède 2 à 3 atomes de carbone, de préférence un co-solvant de polyéthylèneglycol.

26. Procédé selon la revendication 24, dans lequel ladite formulation est telle que définie selon l'une quelconque des revendications 1 à 19.

27. Procédé selon l'une quelconque des revendications 24 à 26, dans lequel une quantité dudit tensio-actif polyoxyalkylène non ionique est incluse dans ledit au moins un solvant alcanol dans lequel la cyclosporine est dissoute, ladite quantité représentant jusqu'à 50 pourcent en poids de la composition utilisée pour dissoudre la cyclosporine.

28. Procédé pour former une dispersion aqueuse de particules de cyclosporine comprenant l'étape consistant à diluer dans un milieu aqueux la formulation produite par le procédé selon l'une quelconque des revendications 24 à 27.

29. Procédé selon la revendication 28, dans lequel ledit milieu aqueux est l'eau.

30. Procédé selon la revendication 28 ou 29, dans lequel au moins 50 pourcent en poids de la cyclosporine présente dans ladite dispersion aqueuse sont faits de particules inférieures à environ 1 µm, ladite cyclosporine étant amorphe.

31. Utilisation d'une dispersion aqueuse de particules de cyclosporine préparées par un procédé selon l'une quelconque des revendications 28 à 30 dans la fabrication d'un médicament en vue du traitement d'un patient nécessitant un traitement immunosuppresseur.
